(19) Europäisches Patentamt
European Patent Office
Office européen des brevets

(11) Veröffentlichungsnummer: **0 140 033 B1**

(12) # EUROPÄISCHE PATENTSCHRIFT

(45) Veröffentlichungstag der Patentschrift: **31.01.90**

(51) Int. Cl.⁴: **A 61 K 7/48,** C 07 C 33/20

(21) Anmeldenummer: **84110446.6**

(22) Anmeldetag: **03.09.84**

(54) **Sebosuppressive kosmetische Mittel, enthaltend Alkoxyaryl-alkanole.**

(30) Priorität: **09.09.83 DE 3332505**

(43) Veröffentlichungstag der Anmeldung: **08.05.85 Patentblatt 85/19**

(45) Bekanntmachung des Hinweises auf die Patenterteilung: **31.01.90 Patentblatt 90/5**

(84) Benannte Vertragsstaaten: **AT BE CH DE FR GB IT LI LU NL SE**

(56) Entgegenhaltungen:
**DE-A- 1 443 429**
**GB-A- 1 569 424**
**US-A- 3 663 716**
**US-A- 4 435 605**

**CHEMICAL ABSTRACTS, Band 99, 1983, Seite 520, Nr. 53342p, Columbus, Ohio, US; BETTARINI, FRANCO et al.: "Acaricide compounds"**
**CHEMICAL ABSTRACTS, Band 100, 1984, Seite 596, Nr. 174437w, Columbus, Ohio, US; & JP - A - 58 50 204**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Möller, Hinrich, Dr., Schumannstrasse 11, D-4019 Monheim (DE)**
Erfinder: **Wallat, Siegfried, Dr., Marie-Curie-Strasse 9, D-4019 Monheim (DE)**

**EP 0 140 033 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft topische, kosmetische Mittel zur Verbesserung des fettigen und unästhetischen Aussehens der Haare und der Haut, welche bestimmte Alkoxyarylalkanole enthalten.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein gesundes Aussehen zu geben. Es wurden zur Bekämpfung der Seborrhoe des behaarten Kopfes kosmetische Pflegemittel mit Schwefel-, Quecksilber- oder Teerzusatz verwendet. Dabei hat sich gezeigt, daß diese bekannten antiseborrhoischen Zusätze bei längerer Anwendung häufig zu nebenwirkungen führen, ohne daß wirklich befriedigende Ergebnisse im Hinblick auf Wirksamkeit und anwendungstechnische Eigenschaften erzielt werden konnten. In der DE-A-2 926 267 werden zur Normalisierung der Fettabsonderung 3,7,11-Trimethyl-2,6,10-dodecatrien-1-ol-Derivate als Zusatz zu kosmetischen Pflegemitteln beschrieben. Es hat sich jedoch gezeigt, daß diese Verbindungen nur eine sehr geringe antiseborrhoische Wirkung besitzen.

Aufgabe der Erfindung ist es, ein kosmetisches Mittel bereitzustellen, welches gegenüber den bekannten Präparaten eine verstärkte Wirkung, ohne nachteilige Folgen auf den menschlichen Körper hat.

Es wurde nun überraschend gefunden, daß bestimmte Alkoxyaryl-alkanole hervorragende antiseborrhoische Effekte auch bei sehr geringer Dosierung aufweisen.

Gegenstand der Erfindung sind sebosuppressive kosmetische Mittel, die gekennzeichnet sind durch einen Gehalt an Alkoxyaryl-alkanolen der allgemeinen Formel

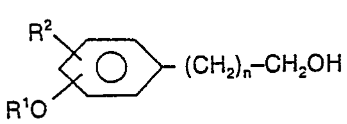

worin bedeuten:

$R^1$ = Alkyl-$C_4$–$C_{20}$, geradkettig oder verzweigt,

$R^2$ = Alkyl-$C_1$–$C_4$, Alkoxy-$C_1$–$C_4$, OH, oder vorzugsweise H,

n = 0 oder 1,

wobei $R^1O$ – die Positionen 2, 3 oder vorzugsweise 4, $R^2$ irgendeine der übrigen Positionen am Ring einnehmen kann.

Die anspruchsgemäß zu verwendenden Verbindungen sind zum Teil bekannt und können nach allgemein bekannten Verfahren hergestellt werden. Soweit sie neu sind, können sie ebenfalls nach an sich bekannten Verfahren erhalten werden. Beispielsweise besteht ein bevorzugtes Herstellungsverfahren in der katalytischen oder chemischen Reduktion, vorzugsweise mit komplexen Hydriden des Aluminiums oder Bors, der entsprechend substituierten Arencarbonsäureester, Arencarbaldehyde bzw. Arylessigsäureester oder Arylacetaldehyde.

Ein anderes Verfahren besteht in der Hydrolyse der entsprechenden Alkoxyarylakylhalogenide. In einigen Fällen ist auch die direkte Alkylierung von Aromaten mit Formaldehyd möglich.

Als erfindungsgemäß einzusetzende Alkoxyarylalkanole sind die durch folgende Gruppen substituierten Benzylalkohole bzw. Phenylethan-2-ole zu nennen:

2-, 3-, 4-Butoxy-, -tert.Butoxy-, -Hexyloxy-, -Heptyloxy-, -Octyloxy-, -(2-Ethylhexyloxy)-, -Nonyloxy-, -Isononyloxy-, -(3,5,5-Trimethyl-hexyloxy-), -Decyloxy-, -Undecyloxy-, -Dodecyloxy-, -Isotridecyloxy-, -Tetradecyloxy-, -(2-Hexyl-decyloxy)-, -Hexadecyloxy-, Octadecyloxy-, -Eicosyloxy-, 4-Dodecyloxy-3-methoxy-, 4-Dodecyloxy-3-hydroxy-, 4-Tetradecyloxy-2-hydroxy-, 2-Dodecyloxy-3-methyl-, 3-Octyloxy-4-ethoxy-, 2-Decyloxy-4-methyl-benzylalkohol und -2-(4-Decyloxyphenyl)-ethanol.

Die Verbindungen besitzen eine ausgeprägte sebosuppressive Wirkung bei ausgezeichneter Haut- und Schleimhautverträglichkeit. Sie lassen sich ohne Schwierigkeit in verschiedene kosmetische Zubereitungen, wie wäßrige oder alkoholische Lösungen, Öle, Suspensionen, Gele, Emulsionen, Salben oder Aerosole einarbeiten. Zur Behandlung von seborrhoischer Haut und fetten Haaren können diese in allen üblichen Applikationsformen wie Haarwässern, Haarshampoos, Haarkuren, Haarspülungen, Hautlotionen oder Schüttelmixturen eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe wie Wasser, organische Lösungsmittel, oberflächenaktive Verbindungen, Öle, Fette, Wachse, Duftstoffe, Farbstoffe, Konservierungsmittel und dergleichen enthalten. Die neuen sebosuppressiven Mittel enthalten zweckmäßig 0,01–5,0 Gew.-%, vorzugsweise 0,05–1,0 Gew.-% der Alkoxyaryl-alkanole.

Herstellungsbeispiele:

A) 4-Tetradecyloxy-benzylalkohol
(neue Verbindung)

In 60 ml Diethylenglykoldimethylether wurden 1,35 g (36 m Mol) Natriumborhydrid und anschließend 3,1 g (36 m Mol) Lithiumbromid eingerührt. Nach 30 Min. Rühren wurden 20,0 g (57 m Mol) 4-Tetradecyloxy-benzoesäuremethylester zugegeben. Die Mischung wurde 3,5 Std. auf 100 °C erwärmt, portionsweise auf ein Gemisch von 500 g Eis und 50 ml konz. Salzsäure gegossen, der gebildete Niederschlag abfiltriert, mit wenig Methylenchlorid verrührt, der ungelöste Teil abfiltriert und aus n-Hexan umkristallisiert. Es wurden 12,5 g 4-Tetradecyloxy-benzylalkohol vom Schmp. 73–75 °C erhalten.

B) 4-Decyloxy-benzylalkohol
wurde analog A) erhalten: Schmp. 55–57 °C.

C) 2-(4-Decyloxy-phenyl)-ethanol (neue Verbindung)
wurde analog A) aus 2-(4-Decyloxy-phenyl)-essigsäuremethylester erhalten: Schmp. 42–44 °C.

D) 4-Dodecyloxy-benzylalkohol (neue Verbindung)
wurde analog A) hergestellt: Schmp. 65–66 °C

E) 4-Hexyloxy-benzylalkohol
wurde analog A) hergestellt: Schmp. 32–33 °C

F) 4-Octyloxy-benzylalkohol
wurde analog A) erhalten: Schmp. 47–48 °C

G) 2-Decyloxy-benzylalkohol
wurde analog A) erhalten: Sdp. 153–156 °C/0,04 m bar

H) 3-Dodecyloxy-benzylalkohol (neue Verbindung)
wurde analog A) erhalten: 44–46 °C

I) 3-Tetradecyloxy-benzylalkohol (neue Verbindung)
wurde analog A) erhalten: Schmp. 50–53 °C

J) 2-Dodecyloxy-benzylalkohol
wurde analog A) erhalten: Sdp. 166 °C/0,03 m bar, n: 1,4968

K) 3-Decyloxy-benzylalkohol (neue Verbindung)
wurde analog A) hergestellt: Schmp. 37–39 °C

L) 4-Octadecyloxy-benzylalkohol (neue Verbindung)
wurde analog A) erhalten: Schmp. 84–87 °C

M) 4-(2-Ethyl-hexyloxy)-benzylalkohol (neue Verbindung)
wurde analog A) erhalten: Sdp. 130 °C/0,15 m bar n: 1,5068

N) 4-(3,5,5-Trimethyl-hexyloxy)-benzylalkohol (neue Verbindung)
wurde analog A) erhalten: n: 1,5025

O) 4-(2-Hexyl-decyloxy)-benzylalkohol (neue Verbindung)
wurde analog A) hergestellt: n: 1,4932

P) 4-Dodecyloxy-3-methoxy-benzylalkohol (neue Verbindung)
wurde analog A) erhalten: Schmp. 55–57 °C

Die antiseborrhoische Wirkung wurde durch nachfolgend beschriebene Tierversuche näher untersucht.

Als Versuchstiere dienten männliche Wistar-Ratten mit einem Körpergewicht von 220–230 g zu Versuchsbeginn. Beurteilt wurde visuell der Bräunungsgrad auf dem Rücken der dort geschorenen Ratten. Die Bräunung wird durch das braune Hautoberflächelipid der Ratten hervorgerufen. Dieser Test geht von der Beobachtung aus, daß junge weibliche Ratten sowie männliche Ratten nach dem Waschen mit Tensidlösung bzw. einem Lipidlösungsmittel und auch männliche Ratten, die systematisch mit Östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren.

Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Bewertungsmethoden:

Als 1. Kriterium wurde bewertet, ob die Mehrheit der Beurteiler richtig die behandelte Seite erkannt haben, wobei wie folgt differenziert wurde:

| Zeichen | Anteil der Beurteiler, die eine Wirkung erkannten |
|---|---|
| ++ | 100% |
| + | >50%–100% |
| 0 | ≤50% |

Als 2. Kriterium wurde der Unterschied zwischen rechter und linker Seite gewertet, wobei pro Beurteiler und Tier jeweils 1 Punkt zu vergeben war, und zwar in der Weise, daß die

| | |
|---|---|
| dunklere Seite | mit 1 Punkt |
| hellere Seite | mit 0 Punkte und |
| Gleichheit beide Seiten | mit 0,5 Punkten |

benotet wurden.

Signifikante Differenzen zwischen unbehandelter und behandelter Seite nach der zweiten Bewertungsmethode zeigen die lokale Wirksamkeit einer Substanz an.

Als 3. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach der dritten Bewertungsmethode werden die Punktsummendifferenzen zwischen den unbehandelten Kontrolltieren und jeweils den behandelten und unbehandelten Seiten der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet:

| Zeichen | Punktdifferenz | |
|---|---|---|
| ++ | sehr groß | (≥99,9% Wahrscheinlichkeit) |
| + | signifikant | (>95% Wahrscheinlichkeit) |
| 0 | minimal | (95% Wahrscheinlichkeit) |

Prozentuale Sebumreduktion

Die Sebumreduktion errechnet sich aus der Punktedifferenz in der Weise, daß man den Quotienten aus der Punktedifferenz $\Delta P$ und der Punktezahl für die Kontrollgruppe $P_k$ bildet und den erhaltenen Wert in % angibt.

$$\text{Sebumreduktion} = \frac{\Delta P}{P_k} \cdot 100\ [\%]$$

Die p-Alkoxy-benzoesäureester wurden in Konzentrationen von 0,1 bzw. 0,2 und 1,0% in Alkohol/Aceton (1 : 1) in der angegebenen Weise appliziert. Die Ergebnisse sind in der Tabelle zusammengefaßt.

Tabelle
Bewertung der sebosuppressiven Effekte

| Substanz | Konz. (%ig) | Bewertungsmethode 1 | 2 | 3 | Sebumreduktion (%) |
|---|---|---|---|---|---|
| A | 0,5 | ++ | ++ | ++ | 97 |
| B | 0,2 | ++ | ++ | ++ | 95 |
| C | 1,0 | ++ | ++ | ++ | 56 |
| D | 0,1 | ++ | ++ | ++ | 88 |
| E | 0,2 | ++ | ++ | ++ | 92 |
| F | 0,2 | ++ | ++ | ++ | 95 |
| G | 0,5 | ++ | ++ | ++ | 20 |
| H | 0,5 | ++ | ++ | ++ | 69 |
| I | 0,5 | ++ | ++ | ++ | 22 |
| K | 0,5 | ++ | ++ | ++ | 53 |
| Benzylalkohol (US-A-3.663.716) | 1,0 | 0 | 0 | 0 | 0 |

Beispiele für kosmetische Rezepturen

Nachfolgend werden für die erfindungsgemäßen topischen Mittel zur Behandlung von stark fettendem Haar und seborrhoischer Haut Rezepturen gegeben:

1. Shampoo für fettendes Haar

| | Gewichtsteile |
|---|---|
| Ammoniumlaurylsulfat mit 33–35% Waschaktivsubstanz (Texapon A®) | 40,0 |
| Kokosfettsäurediethanolamid (Comperlan KD®) | 3,0 |
| Natriumchlorid | 2,0 |
| Natriumsulfat | 2,0 |
| 4-Dodecyloxy-benzylalkohol (D) | 0,5 |
| Konservierungsmittel | 0,1 |
| Parfümöl | 0,1 |
| Wasser | 52,3 |

2. Haarkur

| | Gewichtsteile |
|---|---|
| Glycerinmono-distearat (Tegin M®) | 0,7 |
| kationisches Tensid (Cetyltrimethyl-ammoniumchlorid, Dehyquart A®) | 2,0 |
| Chloesterin | 0,2 |
| Sojalecithin | 0,3 |
| Emulgade A® (Gemische von Cetylstearylalkohol mit nichtionogenen Emulgatoren) | 8,0 |
| Parfümöl | 0,3 |
| 4-Decyloxy-benzylalkohol (B) | 0,2 |
| Wasser, vollentsalzt | 88,3 |

3. Hautcreme

| | Gewichtsteile |
|---|---|
| Selbstemulgierendes Gemisch aus Mono/Diglyceriden höherer gesättigter Fettsäuren mit Kaliumstearat (Cutina KD 16®) | 16,0 |
| Cetylstearylalkohol mit ca. 12 Mol Ethylenoxid Emulgin B 1®) | 1,0 |
| 2-Octyldodencanol | 8,0 |
| Isopropylmyristat | 6,0 |
| Glycerin | 6,0 |
| 4-Octyloxy-benzylalkohol (F) | 0,5 |
| Wasser | 62,5 |

Bezugsquellen für die genannten Handelsprodukte:

| | |
|---|---|
| Texapon A® | = Henkel KGaA |
| Comperlan KD® | = Henkel KGaA |
| Cutina KD 16® | = Henkel KGaA |
| Eumulgin B 1® | = Henkel KGaA |
| Tegim M® | = Atlas Chemie |
| Dehyquart A® | = Henkel KGaA |

**Patentansprüche**

1. Sebosuppressive kosmetische Mittel, gekennzeichnet durch einen Gehalt an Alkoxyarylalkanolen der allgemeinen Formel

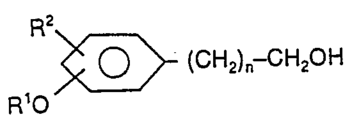

worin bedeuten:

$R^1$ = Alkyl-$C_4$–$C_{20}$, geradkettig oder verzweigt,

$R^2$ = Alkyl-$C_1$–$C_4$, Alkoxy-$C_1$–$C_4$, OH, oder vorzugsweise H,

n = 0 oder 1,

wobei $R^1O$ – die Positionen 2, 3 oder vorzugsweise 4,

$R^2$ irgendeine der übrigen Positionen am Ring einnehmen kann.

2. Mittel nach Anspruch 1, gekennzeichnet durch einen Gehalt von 0,01 bis 5,0 Gew.-%, vorzugsweise 0,05 bis 1,0 Gew.-% an Alkoxyarylalkanolen.

3. Verwendung von Mitteln nach einem der Ansprüche 1 oder 2 zur Herstellung von Arzneimitteln für die Behandlung von fettiger Haut und fettigen Haaren.

**Claims**

1. Sebosuppressive cosmetic preparations, characterized by a content of alkoxyaryl alkanols corresponding to the following general formula

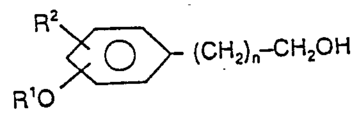

in which

$R^1$ represents a linear or branched $C_1$–$C_{20}$ alkyl radical,

$R^2$ represents a $C_1$–$C_4$ alkyl radical, a $C_1$–$C_4$ alkoxy radical, an OH group or, preferably, H,

n = 0 or 1,

$R^1O$ occupying the 2, 3 or, preferably, 4 positions and

$R^2$ occupying any of the other positions on the ring.

2. Preparations as claimed in Claim 1, characterized by a content of from 0.01 to 5.0% by weight and preferably from 0.05 to 1.0% by weight of alkoxyaryl alkanols.

3. The use of the preparations claimed in claim 1 or 2 for the production of medicaments for treating greasy skin and greasy hair.

**Revendications**

1. Agents cosmétiques sébosuppresseurs, caractérisés en ce qu'ils contiennent des alcoxyarylalcanols de formule générale:

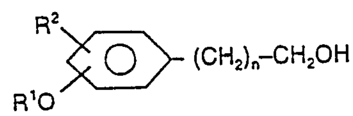

dans laquelle

$R^1$ représente un groupe alkyle en $C_4$–$C_{20}$, à chaîne droite ou ramifiée,

$R^2$ représente un groupe alkyle en $C_1$–$C_4$, un groupe alcoxy en $C_1$–$C_4$, un groupe OH ou, de préférence, H,

n = 0 ou 1,

$R^1O$ pouvant occuper les positions 2, 3 ou, de préférence, 4, tandis que $R^2$ peut occuper l'une quelconque des autres positions sur le noyau.

2. Agents selon la revendication 1, caractérisés en ce qu'ils contiennent 0,01 à 5,0% en poids, de préférence, 0,05 à 1,0% en poids d'un alcoxyarylalcanol.

3. Utilisation d'agents selon une des revendications 1 ou 2, pour la préparation de médicaments destinés au traitement de la peau grasse et des cheveux gras.